# EUROPEAN PATENT SPECIFICATION

(11) **EP 1 159 600 B1**
(45) Date of publication and mention of the grant of the patent: **16.08.2006**
(21) Application number: 99967359.3
(22) Date of filing: 15.12.1999
(51) Int. Cl.: G01N 15/14

(54) **AXIAL PATTERN ANALYSIS AND SORTING INSTRUMENT FOR MULTICELLULAR ORGANISMS EMPLOYING IMPROVED LIGHT SCATTER TRIGGER**
VERBESSERTEN LICHTSTREUUNGSAUSLÖSER VERWENDENDES AXIALMUSTERANALYSE- UND SORTIERGERÄT FÜR VIELZELLORGANISMEN
INSTRUMENT DE TRI ET D'ANALYSE DE MODELES AXIAUX POUR ORGANISMES MULTICELLULAIRES UTILISANT UN DECLENCHEUR DE DISPERSION LUMINEUSE PERFECTIONNE

(30) Priority: 15.12.1998 US 112280 P
(43) Date of publication of application: 05.12.2001
(73) Proprietor: Union Biometrica, Inc., Somerville, MA 02143 (US)
(72) Inventor: HANSEN, Peter, W., Canaan, NY 12029 (US); GERSHMAN, Russell, J., Somerville, MA 02143 (US); KRAULEDAT, Petra, B., New York, NY 12029 (US)
(74) Representative: HOFFMANN EITLE
(86) International application number: PCT/US1999/029909
(87) International publication number: WO 2000/036396

(56) References cited:
- US-A- 4 693 602
- US-A- 4 769 776
- US-A- 5 475 487
- US-A- 5 798 222

## Description

### BACKGROUND OF THE INVENTION

### 1. Field of the Invention

The present application concerns instruments to analyze and separate objects suspended in a fluid-specifically such instruments optimized to analyze and separate elongated multicellular organisms.

### 2. Description of Related Art

The present invention pertains to high-speed mechanisms for automatically identifying and physically selecting multicellular organisms with certain spatially distinct, optically detectable, phenotypic characteristics from mixed populations. Examples of applicable multicellular organisms are all stages of *Caenorhabditis elegans, Drosophila melanogasler* (fruit fly) larvae, or *Danio rero* (zebrafish) embryos. These are useful as model organisms for human disease and functional genomics studies. Examples of spatially distinct, optical characteristics are; the localized expression of DNA encoded fluorescent protein molecules, localized variations of the index of refraction or granularity, or localized variations in specific binding sites (receptors) for optically labeled antibodies, lectins, or other specific ligands.

Intact multicellular organisms, such as C. elegans, *D. melanogaster* larvae, or *D. rero* embryos are frequently used as model systems to help understand the function of human genes that have been implicated in disease. Human gene homologues have been identified in these model organisms and mutations have been induced specifically in those gene homologues. Such mutations frequently result in an easily observable phenotypic change in the model organism and it has been shown that certain mutants respond to pharmacological compounds and these responses collaterally produce optically detectable changes in the organism.

Mutants of intact organisms are now used as a new class of *in vivo* drug screens for libraries of potential pharmacological compound produced through use of combinatorial chemical methods. With these organisms, one can identify targets for drug intervention without the need to completely understand complex biochemical pathways that relate the genome to the phenotype. This allows rapid and economical screenings of the compound libraries for new and useful human drugs while limiting politically controversial testing on mammals.

The exposure of model organism mutants to diverse drug compound libraries, even when the specific mutations involved have not yet been linked to human gene homologues also helps define gene function. The addition of such functional genomic techniques to the repertoire of molecular biology and biochemistry methods can greatly accelerate the drug discovery process. Investigators can annotate drug libraries for toxicity, non-specific activity, or cell membrane permeability by observing their behavior in intact organisms. This way, toxic or ineffective libraries and/or library members can be discarded at an early stage without wasting valuable resources.

While model organisms such as the nematode *C. elegans,* the fruit fly *D. melanogaster,* and the zebrafish *D. rero* have been proven useful in the study of human disease, they have not yet been successfully used in the field of high speed, high throughput drug discovery. Until now high-speed preparation and analysis techniques have been missing for these large organisms. This presents a roadblock to investigators that need to search through thousands of multicellular organisms for a new mutation or for response to a given sample drug. For example, with today's molecular biology techniques, a large laboratory can produce deletion mutations in a multicellular test organism at a rate of 20 to 30 per month. Then, in order to evaluate the effect of a chemical compound library (that frequently contains 100,000 discrete compounds) on a class of mutated organisms, one must first manipulate and deposit a precise number of organisms of the mutant strain and the same development stage into various containers such as wells of a microtiter plate array. Wild type or deviants from the desired mutant strain, or organisms at a different development stage must be eliminated. Using slow, manual methods, the selection and deposition of organisms of the proper type is a bottleneck in time to the entire process of drug discovery. Additionally, manual methods rely on pipettes that dispense accurate volumes of fluid but not accurate numbers of organisms. In many studies where reproduction rate is altered by the mutation, it is necessary to begin the study of the effect of a compound from the combinatorial library with an exact, and known number of multicellular organisms in each well. This is, at best, a daunting requirement.

In addition to the need for rapid preparative methods there is a need for rapid analysis methods. For example, if a mutant strain or expression system can be characterized by a spatial pattern of fluorescence or staining, then the effect of therapeutic compounds or toxic environments on these strains might be determined by changes in these patterns. For example, green fluorescent protein (GFP) is used as reporter gene to indicate that an inserted gene has been expressed. The expression of the fluorescent protein usually occurs in a specific spatial pattern within a multicellular organism. Discrimination of one pattern from another is currently carried out manually with the fluorescent microscope. This is an extremely tedious task requiring a significant number of workers that are trained at very high academic levels.

US 6,400,453 describes an instrumentation system for the rapid analysis and sorting of multicellular organisms using optical characteristics such as light scatter and fluorescence to classify each organism in a flowing stream. A single value of fluorescence intensity at a given emission wavelength is detected and assigned to each organism. The present invention is an improvement that enables a flow analyzer and sorter to localize and report not only the intensity but also the position of fluorescence along the major (long) axis of the organism and use this new spatial information to sort the organisms.

If a mutant strain or transgenic organism is characterized by a stable, spatial pattern of fluorescence, staining or other optically detectable characteristics, then the effect of therapeutic compounds or toxic environments on these strains can potentially be determined by monitoring changes in these spatial patterns. Discrimination of one pattern from another is currently carried out manually with the fluorescent microscope. This is an extremely tedious task requiring a significant number of workers that are trained at very high academic levels. Automating the detection of spatial patterns of fluorescence will improve the objectivity and the speed of measurement.

Flow instruments have been used before to count the number of nematodes in a fluid volume. Such a device was described by Byerly et al (L. Byerly, R.C. Cassada, and R.L. Russell, "Machine for Rapidly Counting and Measuring the Size of Small Nematodes" , *Rev. Sci. Instrum.* Vol. 46, No. 5, May 1975) where the flow cytometer employed sheath flow to orient the nematodes along the direction of flow so that their size could be measured and organism-by-organism counts could be made by an electrical impedance method. The device was similar to a commercial Coulter counter. The present invention differs from the Byerly device in that it can provide a device that selects and deposits (sorts) specific organisms. The present invention is also not limited to using an impedance sensor, which can only estimate overall size, but instead uses optical sensing to spatially resolve localized features along the major axis of the organism and use these to analyze and sort.

An optical flow instrument for analyzing elongate organisms such as plankton with widths of 500 µm and lengths over 1000 µm has been described with sheath flow to achieve orientation of the plankton. (J.C. Peeters, G.B. Dubelaar, J. Ringelberg, and J.W. Visser, "Optical Plankton Analyser: a Flow Cytometer for Plankton Analysis, I: Design Considerations" *Cytometry* 1989 Sept 10 (5): 522-528; and G.B. Dubelaar, A.C. Groenwegen, W. Stokdijk, G.J. van den Engh, and J.W. Visser, "Optical Plankton Analyser: a Flow Cytometer for Plankton Analysis, II: Specifications" , *Cytometry* 1989 Sept 10 (5): 529-539). The size range of the plankton used in these optical flow cytometers is similar to that encountered with *C. elegans* nematodes, fruit fly larvae, and zebrafish embryos; however there is no provision that avoids the ambiguous light scatter signals that are eliminated by the present invention.

### SUMMARY OF INVENTION

The present invention provides an instrument for analyzing and selectively dispensing elongate multicellular organisms comprising a source containing multicellular organisms in a fluid suspension; means for causing the fluid suspension to move in a direction of flow; means for aligning the elongate multicellular organisms relative to the direction of flow; a light source for producing an optical beam through which the elongate multicellular organisms pass after becoming aligned; a first optical detector for detecting light over a solid angle of at least 0.03 π steradians (20 degree) scattered by the elongate multicellular organisms for detecting a passage of said organisms through the optical beam; at least one additional optical detectors for detecting sequential optical characteristics arrayed along a length of the multicellular organism, means for creating a data representation of the sequential optical characteristics, means for analysing said data representation, and a fluid switch downstream of a point where said organisms pass through said optical beam, said switch adapted to respond to the means for analyzing to allow detected objects to pass to a sample container.

In one embodiment the output of one optical detector is used to gate the output of other optical detectors.

In a further embodiment, the means for creating a data representation of sequential optical characteristics is adapted to operate upon the gated outputs of the additional detectors.

In a further embodiment, the instrument comprises a controller connected to the fluid switch operative to cause said switch to select multicellular organisms having data representations meeting predetermined criteria.

A method of selectively dispensing elongate multicellular organisms comprising the steps of centering and orienting the sample objects in a flowing fluid stream; passing the fluid stream through a sensing zone, optically detecting the presence of a multicellular organism passing through the sensing zone by means of a light scatter sensor that has an solid acceptance angle of at least 0.03 π steradians (20 degrees), gating output signals from additional optical sensors with an output of the light scatter sensor, creating a data representation of sequential optical characteristics of the multicellular organism comprising output signals from the additional optical sensors; analysing the data representation to select a detected organism and controlling a fluid switch to allow detected objects to pass to a sample container.

In one embodiment, the detected organism is passed to a test chemical or a test environment.

In a further embodiment, the multicellular organisms are exposed to a test chemical or a test environment prior to passing through the optical sensing zone to determine whether the data representation is altered by the test chemical or the test environment.

The present invention uses a fluid flow stream to orient elongate, multicellular organisms and a narrowly focused, stationary, optical beam to scan them along their major axis as the flow. Features such as cell density, refractility granularity, and fluorescence can be detected and recorded as a function of position along the length of the oriented organism (*i.e*., an axial pattern scan). The invention is an improvement in speed and statistical precision over current manual techniques for analyzing multicellular organisms one by one under the microscope. The information from the scan can be used to characterize gene expression and enable physical selection and deposition of phenotypes with desired characteristics, or it can be used to determine alterations in gene expression caused by toxic or therapeutic compounds.

In the case where fluorescence from these organisms is very weak, comparatively high levels of electronic noise accompany the electronic signals that are generated by the fluorescence detector and its associated circuitry. These weak signals cannot be used to mark the presence of an organism, and another, less noisy, signal must be used to gate fluorescence detection. Axial light loss might be used as such a gate. Another preferred gate can be derived from the low-noise light scatter signal from the organism. Conventional light scatter gating, such as is practiced in flow cytometry of single cells, creates ambiguous signals when used on multicellular organisms and thus leads to false gating of fluorescence. A light scatter detection means is herein described which unambiguously gates these fluorescence signals. These signals can then be correlated with position along the major axis of elongate, multicellular organisms and used as enhanced analysis and sorting parameters.

Traditional optical flow cytometers analyze and sort small particles and single cells in liquid suspension by detecting light scatter within (over) narrow cone or solid angles at various angles to the incident optical beam and fluorescence emission at various wavelengths. Information about cell size and structure can be derived from light scatter collected at different angles. For example, information about size can be derived from light scatter detected at low angles relative to the incident optical beam while information about internal cellular granularity can be derived from light scatter detected at a wide angle (near a right angle) relative to the optical beam. Further, the prior art shows that size of the granular structures to be detected determines the angle and acceptance cone for optimal wide-angle detection.

Light scatter signals collected at specific angles and over narrow cone angles are also used to gate detectors of weak fluorescence from single cells. Weak fluorescence signals cannot be effectively used to mark the presence of a cell in the optical beam because high levels of electronic noise accompany these signals. Noise spikes frequently exceed the threshold level for fluorescence detection and produce false readings that are confused as weakly fluorescing cells. To avoid this, flow cytometers generally use signals from one or more detectors situated to detect light scatter at one or more angles relative to the beam to produce relatively noise free signals that can effectively discriminate against false fluorescence from electronic noise, and gate true fluorescence from cells (The reader attention is drawn to U.S. Patent 4,284,412).

It is key to the use of these light scatter detectors as fluorescence gates that their solid angle of detection be narrow. For example, so-called "low angle forward scatter" (LAFS) detectors are frequently placed as close as 0.5 degrees to the optical axis and collect light only within a one degree cone. Wide-angle light scatter detectors are frequently placed at positions ranging from approximately 10 degrees to 90 degrees off axis and also collect light within small cone angles of less than five degrees. If the cone angle of collection is not kept as small as possible, then information about granularity and size can become merged. Under these conditions for example, large cells become indistinguishable from small cells and granular cells become indistinguishable from non-granular cells of the same size.

When narrow acceptance cone light scatter (NACLS) detectors are used to monitor the passage of multicellular organism such as *C*. *elegans,* three problems arise that do not occur with single cells such as blood cells. First, it is found that the light scatter signal does not necessarily rise above baseline (zero) at the beginning of the passage of the organism through the optical beam, but instead rises at an unpredictably later time. Second, it is found that the light scatter signal does not necessarily return to baseline (zero) at the end of the passage of the organism through the optical beam, but instead returns at an unpredictably early time. Third, it is also found that the light scatter signal frequently returns to baseline (zero) at one or more unpredictable times while the organism is in the beam.

Therefore, the most basic effort to size multicellular organisms based on their "time of flight" through the analysis light beam is thwarted by this unpredictable behavior of light scatter signals that are collected over narrow cone angles. Furthermore, the narrow cone angle light scatter signals that start late are not useful for gating weak fluorescence signals. Finally, the narrow cone angle light scatter signals that return to baseline early cannot be used to denote the position of weak fluorescence along the axis of the worm. The signals that return to baseline early can also be confused with the passage of two or more separate organisms when actually only one passed through the analysis beam.

The present invention does not employ the usual single cell, light scatter detection methods, and instead uses light scatter collection over very wide cone angles when analyzing and sorting multicellular organisms. One aspect of the invention is to collect scattered light over a wide solid angle such as 0.03π steradians (20 degrees) or more. This provides a light scatter signal that becomes positive accurately at the time the organism enters the beam, remains unambiguously above baseline while the organism is in the beam, and returns to baseline accurately at the time the organism exits the beam. This aspect of the invention enables another aspect of the invention, which is to use accurate, unambiguous, light scatter signals collected over wide cone angles to mark the linear position of weak and noisy fluorescence signals along the axis of the organism. The width of the cone angle needed depends upon the type of organism.

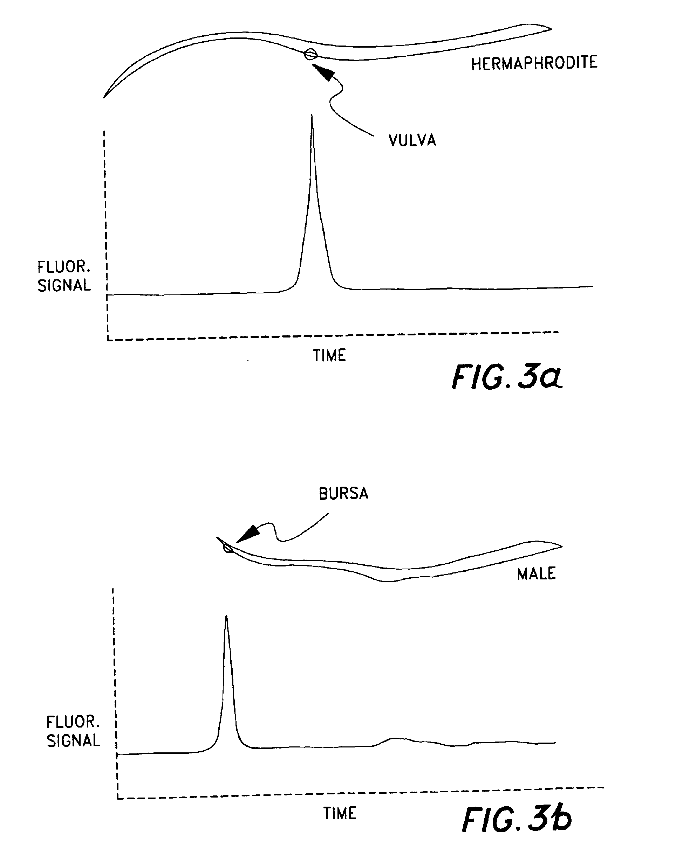

The present invention uses the unambiguous light scatter signal from a wide acceptance angle, light scatter (WACLS) detector as a gate and a timing method for the analysis of fluorescence along the axis of the organism. The location of fluorescence along the axis of the organism is an important parameter for analysis and sorting. For example, with *C. elegans,* it is important in many cloning applications to separate males from hermaphrodites. This can be accomplished with a fluorescently labeled lectin (wheat germ agglutinin) that binds to the vulva of the hermaphrodite and the copulatory bursa of the male. These two structures are not easily distinguishable in brightness, but the vulva is located near the midpoint of the organism and the copulatory bursa is located in the tail. Thus, axial location of fluorescence becomes the parameter for differentially analyzing and sorting males and hermaphrodites. This is illustrated schematically in Figure 3 where two oscilloscope traces are shown for single organisms. One trace (Fig. 3A) has a fluorescent peak near the midpoint, and the other (Fig. 3B) has a fluorescent peak at the tail.

Since there is no fluorescent signal to mark the beginning of the organism in the oscilloscope traces of Figure 3, a means must be established to mark the beginning and end of the passage of the organism through the light beam. This is done by the use of the wide acceptance cone, light scatter (WACLS) signal. The start of this signal triggers a clock in the electronic processor that, in turn causes fluorescent data to be sampled at regular intervals in time while the wide acceptance cone, light scatter signal remains above a preset threshold level. Sampling stops when the WACLS signal drops below threshold, denoting the end of the organism.

The following is a parametric representation of a multicellular organism that can be employed through the use of a WACLS signal to gate the sampling of fluorescence along the organism's axis. Consider a WACLS detector that produces signal S1 and a timing mechanism that samples signals from all other detectors every T microseconds. Assume that there are other light scatter or light absorption detectors situated at various angular positions with respect to the analysis beam. Let the signals from these detectors be denoted by S2, S3, ... Sn. Further assume that there are fluorescence detectors sensitive to various emission wavelengths producing signals F1, F2, F3, ... Fn. The matrix below has columns of data for each detector and rows of data for each sampling interval.

| | S1 | S2 | S3 | ... | Sn | F1 | F2 | F3 | ... | Fn |
|---|---|---|---|---|---|---|---|---|---|---|
| T1 | 0 | 0 | 0 | | 0 | 0 | 0 | 0 | | 0 |
| T2 | a1 | 0 | c1 | | 0 | e2 | 0 | 0 | | 0 |
| T3 | a2 | b2 | c2 | | d3 | 0 | f3 | g3 | | 0 |
| T4 | a3 | 0 | c3 | | d4 | 0 | f4 | 0 | | 0 |
| T5 | a4 | b4 | 0 | | 0 | 0 | f5 | 0 | | 0 |
| Tn-1 | an-1 | 0 | 0 | | 0 | 0 | 0 | gn-1 | | 0 |
| Tn | 0 | 0 | 0 | | 0 | 0 | 0 | | | 0 |

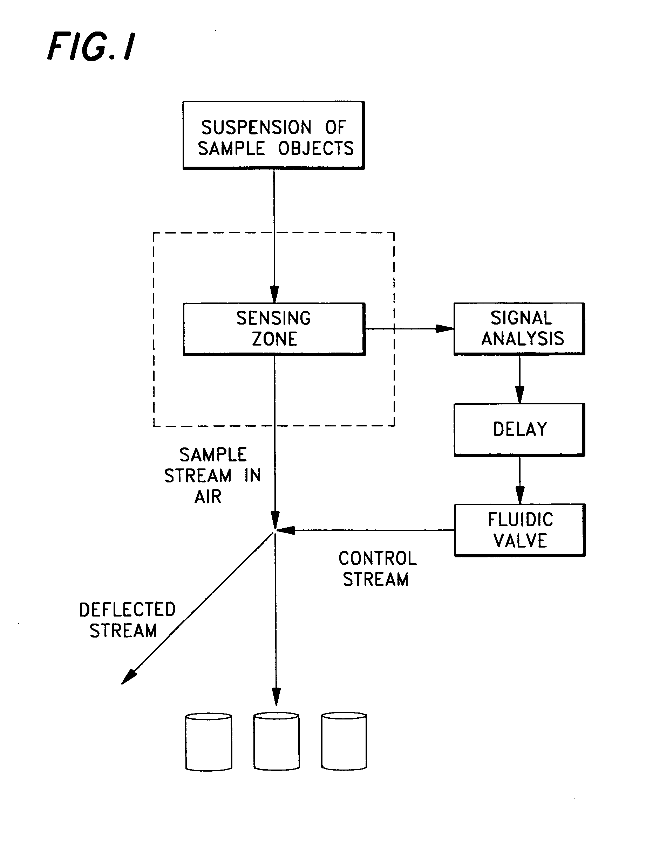

The matrix example above shows a WACLS signal S1 with non-zero entries from time intervals T2 to Tn-1. This is the independent timing signal for all other detector channels. The other light scatter detectors S2 to Sn are not necessarily WACLS detectors, and therefore have zero values during the time T2 to Tn-1. The fluorescence feature with emission wavelength F1 is small and localized within interval T2. This represents a feature that can be used to mark the "tail" of the organism (see Fig. 1).

The fluorescence feature with emission wavelength F2 is not as small (along the axial direction) and occurs at a different location than the F1 feature. The relative location of the feature is established by reference to the timing initiated by the WACLS detector signal S1. If the velocity of the organism is known and the "tail" marker is used, then the absolute location of this feature can be determined as well. The fluorescence feature with emission wavelength F3 shows up in two small locations indicated in the WACLS timing sequence as T3 and Tn-1.

Each scanned organism can be represented by a parametric matrix of this kind. While not containing as much information as a microscope image of the organism, the data acquisition times for such matrices are of the order of five microseconds to 250 microseconds, depending on the length of the organism. This high speed is achieved because simple, fast photomultipliers collect the scattered light and no image is formed. In cytometers images are usually stored by CCD cameras, which are inherently less sensitive than photomultipliers, and therefore require more time to collect enough photons to form an image. Imaging times for fluorescence analysis of organisms such as *C*. *elegans* are of the order of 50 milliseconds, which is from 200 to 10,000 times slower than the time required to collect and store the parametric data described above. The sampling time and the speed of the organism determine the spatial resolution of the parametric method. For example, when the organism typically travels at about 500 cm/sec through the analysis beam, then for a five microsecond sampling time the spatial resolution is approximately 25 µm.

### BRIEF DESCRIPTION OF THE DRAWINGS

Figure 1 shows a diagrammatic representation of optics, flow cell, command electronics, and fluid switch.
Figure 2 shows a diagrammatic representation of the optical beams of the instrument of Figure 1
Figures 3A and 3B show diagrams relating fluorescence signals (gated by one of the methods of the invention) related to hermaphroditic (Fig. 3A) and male (Fig. 3B) *C. elegans* as measured by the instrument of the present invention.
Figure 4A shows an actual oscilloscope traces from a NACLS (lower trace) forward light scatter detector placed at a 45 degree forward light scatter angle and fraction of a degree below the to the optical axis and a fluorescence detector (upper trace) at right angles to the optic axis.
Figure 4B shows an actual oscilloscope traces from a NACLS (lower trace) forward light scatter detector placed at 45 degrees relative to the optical axis and a fluorescence detector (upper trace) placed at right angles relative to the optic axis.
Figure 5 shows actual oscilloscope traces from an extinction detector (lower trace)placed on the optical axis and a fluorescence detector at right angles to the optical axis (upper trace).
Figure 6A shows actual oscilloscope traces from a WACLS forward light scatter detector (lower trace) and a fluorescence detector at right angles to the optical axis (upper trace); the *C*. *elegans* samples scanned showed several discreet points of fluorescence.
Figure 6B shows actual oscilloscope traces from a WACLS forward light scatter detector (lower trace) and a fluorescence detector at right angles to the optical axis (upper trace); the *C*. *elegans* specimens scanned showed a small additional fluorescence at one end..

### DETAILED DESCRIPTION OF THE PREFERRED EMBODIMENTS

The following description is provided to enable any person skilled in the art to make and use the invention and sets forth the best modes contemplated by the inventor of carrying out his invention. Various modifications, however, will remain readily apparent to those skilled in the art, since the general principles of the present invention have been defined herein specifically to provide optical gating devices and methods for use with an optical analyzer/sorter designed for elongated multicellular organisms.

### The flow scanning experimental system

An instrument such as that shown schematically in Fig. 1 was constructed with an interchangeable pair of lasers (argon ion and helium-neon) as the light source. Detection was carried out variously with silicon photodetectors and photomultipliers. The flow cell was rectangular with a square cross-section capillary measuring 250 µm on a side for use with *C. elegans.* The flow cell capillary was 1000 µm on a side to accommodate first through third instar, *D. melanogaster* larvae. Sheath flow is used to orient these elongate organisms as they emerge from the sample nozzle and enter the flow cell capillary.

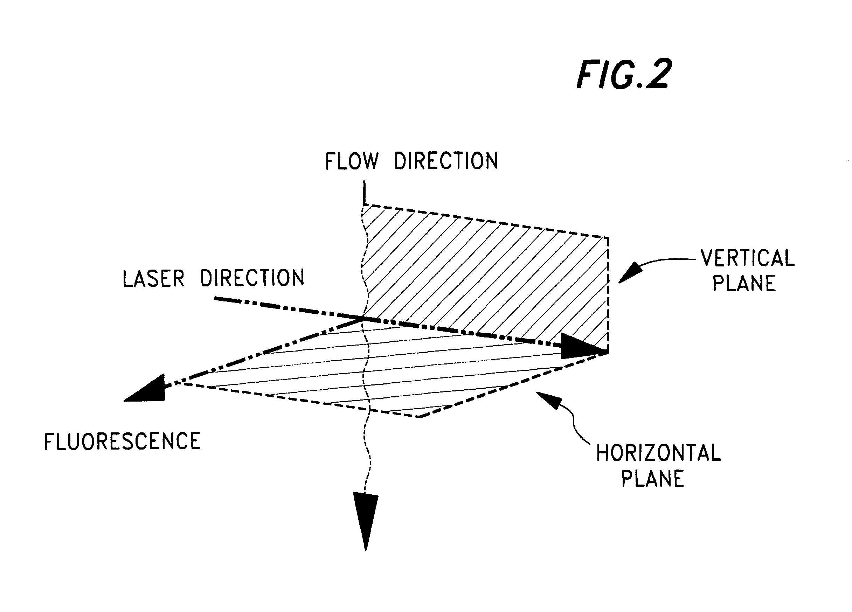

This capillary flow cell is located at the line focus of the laser beam. Fig. 2 diagrammatically shows the geometric relationship of the flow and the various optical beams. The fluorescent light is collected by simple aspheric lenses or microscope objectives and passed through emission filters to photomultipliers. By virtue of the focused laser beam and the collection lenses, the flowing organism is optically scanned as it passes through the focus.

### Simultaneous NACLS and fluorescence from C. elegans

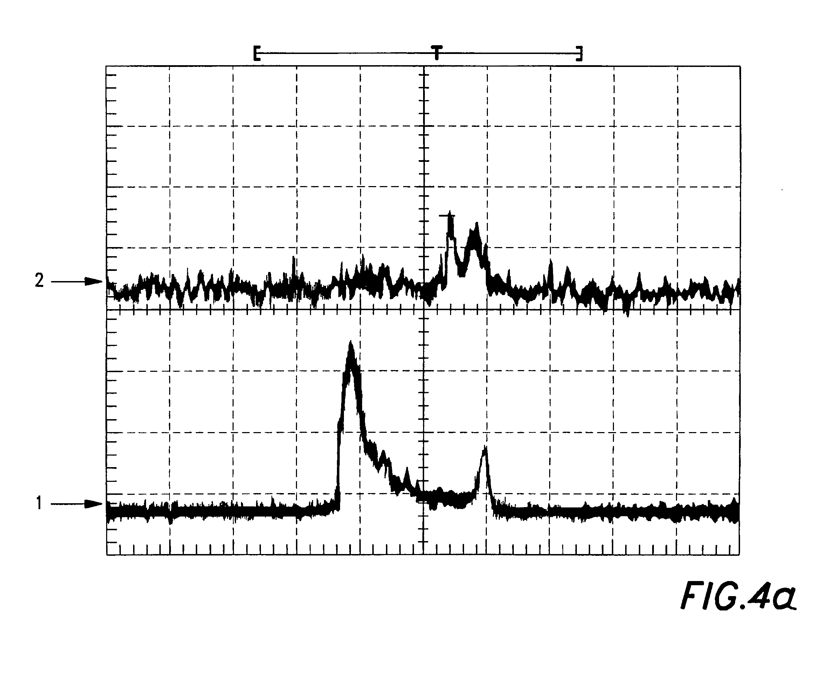

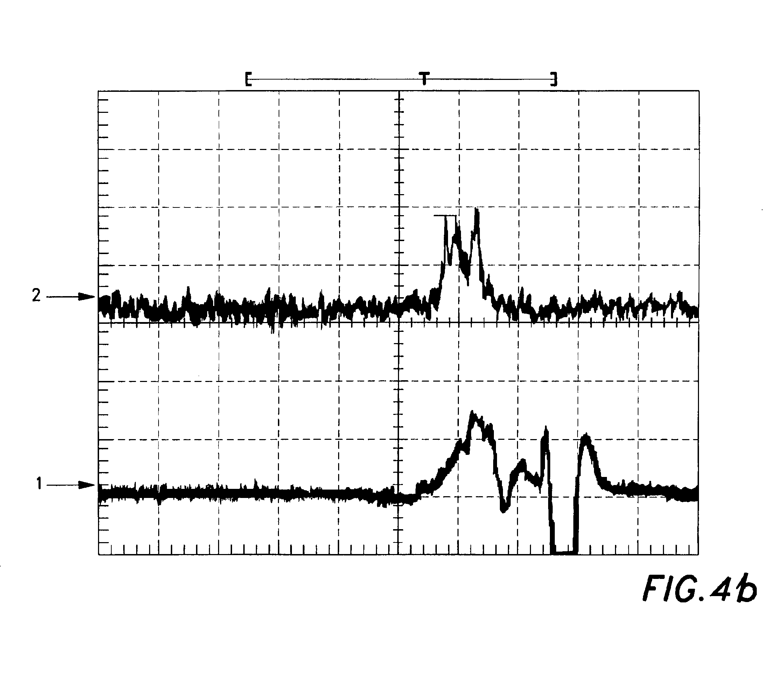

A light scatter sensor was placed at various angular positions with respect to the optical axis in the forward scatter direction. The collection cone angle was approximately six degrees (NACLS). A photomultiplier with a 20X-collection lens and a barrier filter optimized for fluorescence from GFP was used on the fluorescence detector. The *C*. *elegans* that were used for this illustration expressed GFP at two locations in the "head" and nowhere else. The oscilloscope traces for light scatter and fluorescence are shown in Figs 4A and 4B.

The traces show the passage of the organism through the line focus laser beam. The lower trace 1 is the light scatter signal and the top trace 2 is the fluorescence signal. The x-axis is time. Figure 4A is typical of a class of light scatter trace observed with a NACLS detector. The detector was placed at a 45 degree forward light scatter angle directly below the laser beam axis (below the horizontal plane in Fig. 2) as it emerged from the flow cell. No scattered light from the flow cell structures themselves was incident on the detector. The NACLS signal appears to rise at the proper time. The onset of the NACLS trace and the weak autofluorescence trace from the anterior structures of the nematode coincide. The NACLS signal appears to return to baseline after the fluorescent head passes. Unfortunately, the trace returns to baseline approximately during the middle of the passage of the nematode as well. This would give the false impression that two organisms had passed rather than one. This NACLS signal demonstrates the need for a new, unambiguous trigger and timing signal.

Figure 4B illustrates another problem associated with improper placement of a light scatter detector for triggering. In this example, the same detector was placed in the horizontal plane of Fig. 2, but at an angle of 45 degrees to the forward direction. In this case, stray, scattered light from the capillary was incident on the detector. A baseline restoration circuit was used to zero out this light level. The NACLS trace shows a false return to baseline that is caused by the acceptance cone angle being too small, and in addition a place where the signal becomes negative. The negative going region is caused when stray light from the flow cell is blocked by the nematode to an extent that there is more light blockage than there is light scatter. This signal could not be used as a trigger or timing signal for two reasons. The first is that the detector acceptance cone was too small and the second was that stray light on the detector became blocked by the passage of the nematode.

### Problems associated with optical extinction signals as trigger and timing signals

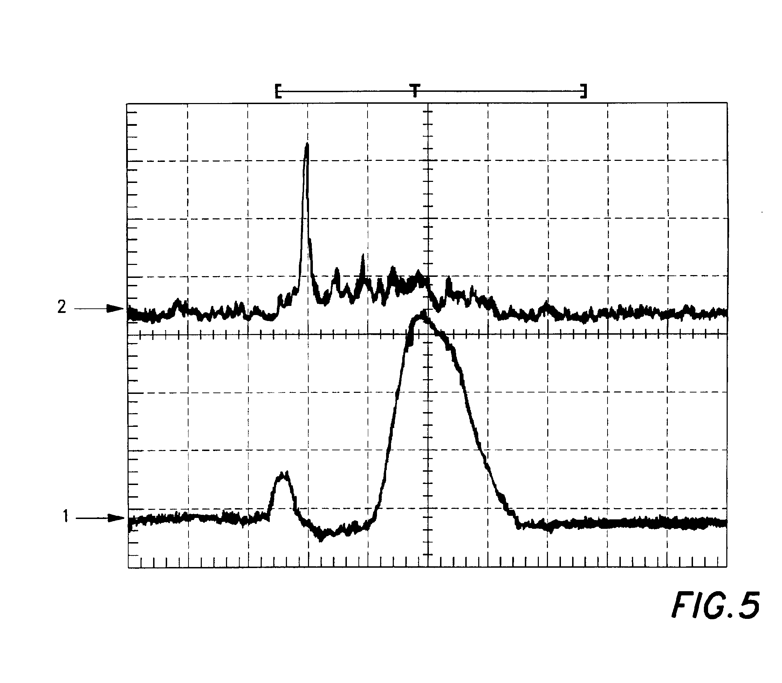

Fig. 5 illustrates another problem associated with improper placement of a light scatter detector for triggering. In this case a sensor was place directly on axis and in the laser beam. The object was to measure light blockage (extinction) by the organisms. Light extinction is a possible alternative to the preferred WACLS trigger of the present invention. The test *C*. *elegans* had a single weak region of fluorescence at a neuronal location in the head located slightly posterior to the tip of the "nose". A 40X objective was used to collect more light since this organism was very weakly fluorescent. The extinction sensor collected light over a two degree cone. In this case extinction trace returns to baseline during the passage of the nematode, and even becomes slightly negative. Therefore, this signal could not be used as a trigger or timing signal.

### Simultaneous WACLS and fluorescence from C. elegans

A photodetector was placed on the optic axis with a collection solid angle of approximately 0,07 π steradians (30 degrees) (WACLS). A mask was placed over the center front of the detector to block any directly transmitted light or stray scattered light from the flow cell capillary. This way, the detector collected light scatter from the organisms over a several times wider cone angle than in the previous examples. The photomultiplier with a 40X collection lens and a barrier filter for green fluorescence protein was used to detect fluorescence since the fluorescence signal was very weak.

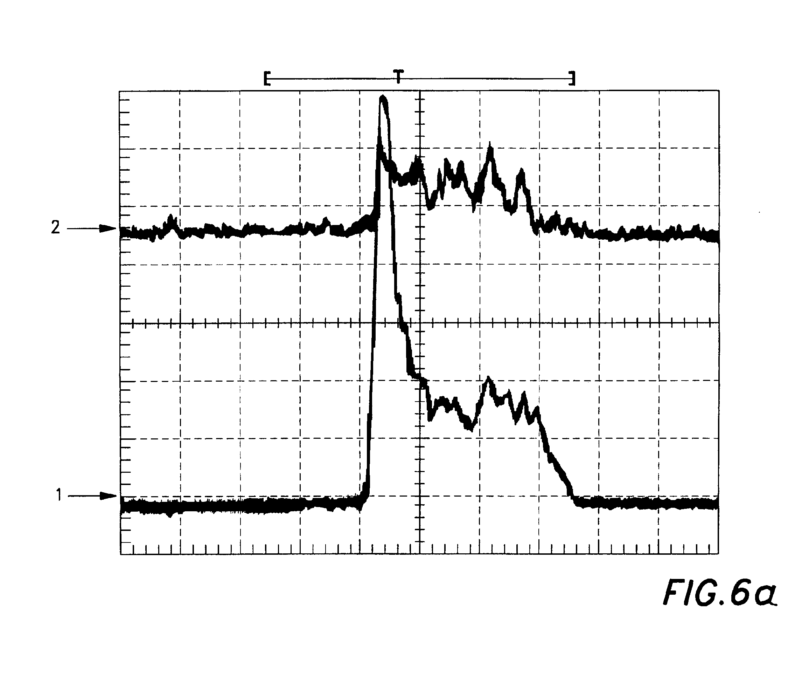

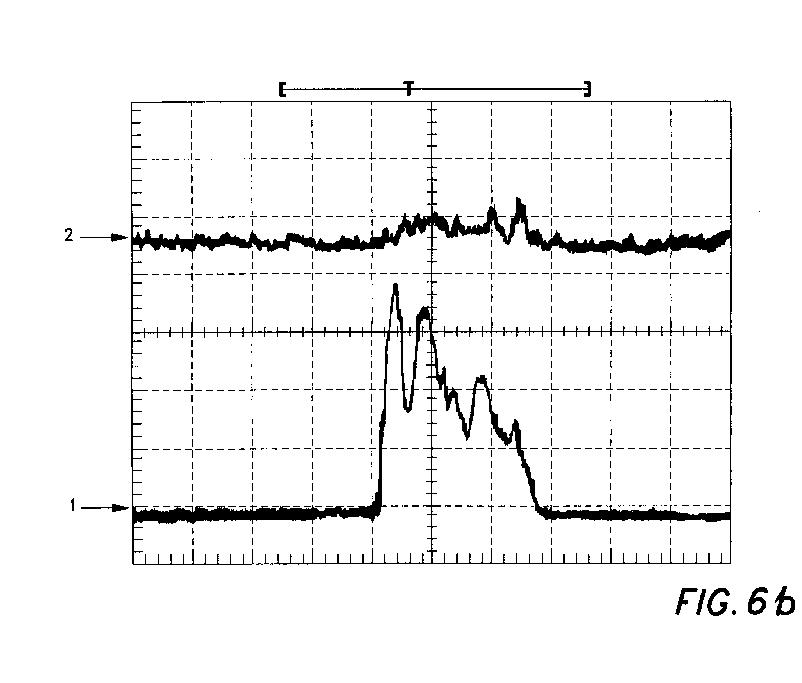

Figure 6A shows a WACLS signal on the lower trace and the associated fluorescence signal on the upper trace. Note that the WACLS signal begins and ends at the proper time and does not return to baseline during the passage of the nematode. This was a consistent and systematic observation so long as the acceptance angle was sufficiently wide and light from the illuminating beam or the scatter detector did not collect stray light. The particular *C*. *elegans* used for this example expressed fluorescence along its entire length with 5 to 6 points along the axis where the expression was locally stronger. Some evidence for these local peaks can be seen in the fluorescence trace. The WACLS signal begins and ends at the proper time and does not return to baseline during the passage of the nematode through the laser beam. There were no exceptions to this observation when over 500 nematodes were analyzed. In the examples of useless trigger signals described above almost half of the signals returned to baseline improperly.

Fig. 6B also shows the traces for a *C*. *elegans* with very weak fluorescent protein expression. There is a low level of autofluorescence throughout the length of the organism and two local regions of weak expression near the tail. The WACLS signal begins and ends at the proper time and does not return to baseline during the passage of the nematode through the laser beam. The fluorescence signal is far too noisy to serve as a self trigger and timing signal, however the onset and end of the WACLS signal is strong and unambiguous, and could be used to time and guide an analysis of the fluorescence trace to the location of the two weak peaks.

In addition to the equivalents of the claimed elements, obvious substitutions now or later known to one with ordinary skill in the art are defined to be within the scope of the defined elements. The claims are thus to be understood to include what is specifically illustrated and described above, what is conceptually equivalent, what can be obviously substituted and also what essentially incorporates the essential idea of the invention. The illustrated embodiment has been set forth only for the purposes of example and that should not be taken as limiting the invention. Therefore, it is to be understood that, within the scope of the appended claims, the invention may be practiced other than as specifically described herein.

## Claims

1. An instrument for analyzing and selectively dispensing elongate multicellular organisms comprising:
a source containing multicellular organisms in a fluid suspension;
means for causing the fluid suspension to move in a direction of flow;
means for aligning the elongate multicellular organisms relative.to the direction of flow;
a light source for producing an optical beam through which the elongate multicellular organisms pass after becoming aligned;
a first optical detector for detecting light over a solid angle of at least 0.03 π steradians (20 degree) scattered by the elongate multicellular organisms for detecting a passage of said organisms through the optical beam;
at least one additional optical detector for detecting sequential optical characteristics arrayed along a length of the multicellular organism,
means for creating a data representation of the sequential optical characteristics, and
means for analysing said data representation,
**characterized by**
a fluid switch downstream of a point where said organisms pass through said optical beam, said switch adapted to respond to the means for analyzing to allow detected objects to pass to a sample container.

2. The instrument of Claim 1, wherein the output of one optical detector is used to gate the output of other optical detectors.

3. The instrument of Claim 2, wherein the means for creating a data representation of sequential optical characteristics is adapted to operate upon the gated outputs of the additional detectors.

4. The instrument of Claim 3, further comprising a controller connected to the fluid switch operative to cause said switch to select multicellular organisms having data representations meeting predetermined criteria.

5. A method of selectively dispensing elongate multicellular organisms comprising the steps of
centering and orienting the sample objects in a flowing fluid stream;
passing the fluid stream through a sensing zone,
optically detecting the presence of a multicellular organism passing through the sensing zone by means of a light scatter sensor that has an solid acceptance angle of at least 0.03 π steradians (20 degrees),
gating output signals from additional optical sensors with an output of the light scatter sensor,
creating a data representation of sequential optical characteristics of the multicellular organism comprising output signals from the additional optical sensors;
**characterized by**
analysing the data representation to select a detected organism and
controlling a fluid switch to allow detected objects to pass to a sample container.

6. The method of Claim 5, wherein the detected organism is passed to a test chemical or a test environment.

7. The method of Claim 5, further comprising the step of exposing the multicellular organisms to a test chemical or a test environment prior to passing through the optical sensing zone to determine whether the data representation is altered by the test chemical or the test environment.

## Patentansprüche

1. Instrument zur Analyse und selektiven Abgabe von länglichen, mehrzelligen Organismen, umfassend:
eine Quelle, die mehrzellige Organismen in einer Fluidsuspension enthält;
Mittel zum Verursachen der Bewegung der Fluidsuspension in einer Flussrichtung;
Mittel zum Ausrichten der länglichen, mehrzelligen Organismen relativ zur Flussrichtung;
eine Lichtquelle zur Erzeugung eines optischen Strahls, durch welchen die länglichen, mehrzelligen Organismen nach deren Ausrichtung durchtreten;
einen ersten optischen Detektor zum Erfassen von Licht, welches von den länglichen, mehrzelligen Organsimen gestreut wurde, über einen Raumwinkel von mindestens 0,03 π Steradiant (20°) um einen Durchtritt der Organismen durch den optischen Strahl zu erfassen;
Mindestens einen zusätzlichen Detektor zum Erfassen sequentieller optischer Eigenschaften, die entlang einer Länge der mehrzelligen Organismen angeordnet sind,
Mittel zur Erzeugung eienr Datendarstellung der sequentiellen optischen Eigenschaften, und
Mittel zur Analyse der Datendarstellung,
**gekennzeichnet durch**
einen Fluidschalter stromabwärts von einem Punkt, wo die Organismen **durch** den optischen Strahl durchtreten, wobei der Schalter geeignet ist, um auf die Mittel zur Analyse zur reagieren und es so den erfassten Objekten zu erlauben, zu einem Probenbehälter zu gelangen.

2. Instrument nach Anspruch 1, wobei die Ausgabe eines optischen Detektors verwendet wird, um die Ausgabe der anderen optischen Detektoren anzusteuern.

3. Instrument nach Anspruch 2, wobei das Mittel zur Erzeugung einer Datendarstellung der sequentiellen optischen Eigenschaften geeignet ist, um in Funktion der angesteuerten Ausgaben der zusätzlichen Detektoren zu arbeiten.

4. Instrument nach Anspruch 3, weiter eine Steuerung umfassend, die mit dem Fluidschalter verbunden ist und arbeitet, um den Schalter zu veranlassen, mehrzellige Organismen auszuwählen, deren Datendarstellungen vorbestimmte Kriterien erfüllen.

5. Verfahren zum selektiven Ausgeben von länglichen, mehrzelligen Organismen, umfassend die folgenden Schritte:
Zentrieren und Orientieren der Probenobjekte in einem fließenden Fluidstrom;
Durchführen des Fluidstroms durch eine Messzone,
Optisches Erfassen des Vorhandenseins eines mehrzelligen Organismus, der durch die Messzone durchtritt, mittels eines Lichtsteuerungssensors, der einen Akzeptanz-Raumwinkel von mindestens 0,03 π Steradiant (20°) aufweist,
Ansteuern der Ausgabesignale von zusätzlichen optischen Sensoren mit einer Ausgabe des Lichtstreuungssensors;
Erzeugen einer Datendarstellung von sequentiellen optischen Eigenschaften der mehrzelligen Organismen, welche Ausgabesignale von den zusätzlichen optischen Sensoren umfasst;
**gekennzeichnet durch**
Analysieren der Datendarstellung, um einen erfassten Organismus auszuwählen und
Steuerung eines Fluidschalters, um es den erfassten Objekten zu erlauben, zu einem Probenbehälter zu gelangen.

6. Verfahren nach Anspruch 5, wobei der erfasste Organismus einer Testchemikalie oder einer Testumgebung zugeführt wird.

7. Verfahren nach Anspruch 5, weiter umfassend den Schritt des Aussetzens des mehrzelligen Organismus an eine Testchemikalie oder eine Testumgebung vor dem Durchführen durch die optische Messzone, um zu bestimmen, ob die Datendarstellung von der Testchemikalie oder der Testumgebung verändert wird.

## Revendications

1. Instrument destiné à analyser et distribuer sélectivement des organismes multicellulaires allongés comprenant
une source contenant des organismes multicellulaires dans une suspension fluide
un moyen destiné à faire déplacer la suspension fluide dans une direction d'écoulement ;
un moyen destiné à aligner les organismes multicellulaires allongés par rapport à la direction de l'écoulement ;
une source de lumière destinée à produire un faisceau optique à travers lequel les organismes multicellulaires allongés passent après avoir été alignés ;
un premier détecteur optique destiné à détecter une lumière sur un angle solide d'au moins 0,03 n stéradian (20 degrés) dispersée par les organismes multicellulaires allongés pour détecter un passage desdits organismes à travers le faisceau optique ;
au moins un détecteur optique supplémentaire destiné à détecter les caractéristiques optiques séquentielles déployées sur une longueur de l'organisme multicellulaire,
un moyen destiné à créer une représentation de données des caractéristiques optiques séquentielles, et
un moyen destiné à analyser ladite représentation de données, **caractérisé par**
un aiguillage de fluide en aval d'un point où lesdits organismes passent à travers ledit faisceau optique, ledit aiguillage adapté pour répondre au moyen destiné à analyser pour laisser les objets détectés passer vers un récipient d'échantillon.

2. Instrument de la revendication 1, dans lequel la sortie d'un détecteur optique est utilisée pour déclencher la sortie d'autres détecteurs optiques.

3. Instrument de la revendication 2, dans lequel le moyen destiné à créer une représentation de données de caractéristiques optiques séquentielles est adapté pour fonctionner sur les sorties déclenchées des détecteurs supplémentaires.

4. Instrument de la revendication 3, comprenant en outre un régulateur connecté à l'aiguillage de fluide fonctionnel pour faire choisir au dit aiguillage les organismes multicellulaires ayant des représentations de données satisfaisant des critères prédéterminés.

5. Procédé de distribution sélective d'organismes multicellulaires allongés comprenant les étapes de
centrer et orienter les objets échantillon dans un courant de fluide s'écoulant ;
passer le courant de fluide à travers une zone de détection,
détecter optiquement la présence d'un organisme multicellulaire passant à travers la zone de détection au moyen d'un capteur de diffusion lumineuse qui possède un angle d'admission solide d'au moins 0,03 n stéradian (20 degrés),
déclencher les signaux de sortie provenant des capteurs optiques supplémentaires avec une sortie du capteur de diffusion lumineuse,
créer une représentation de données des caractéristiques optiques séquentielles de l'organisme multicellulaire comprenant les signaux de sortie provenant des capteurs optiques supplémentaires ;
**caractérisé par**
analyser la représentation de données pour choisir un organisme détecté et
réguler un aiguillage de fluide pour permettre à des objets détectés de passer vers un récipient d'échantillon.

6. Procédé de la revendication 5, dans lequel l'organisme détecté est passé vers un produit chimique de test ou un environnement de test.

7. Procédé de la revendication 5, comprenant en outre l'étape d'exposer les organismes multicellulaires à un produit chimique de test ou un environnement de test avant de passer à travers la zone de détection optique pour déterminer si la représentation de données est altérée par le produit chimique de test ou l'environnement de test.
